# EUROPÄISCHE PATENTSCHRIFT

(11) **EP 0 471 707 B1**
(45) Veröffentlichungstag und Bekanntmachung des Hinweises auf die Patenterteilung: **02.03.1994**
(21) Anmeldenummer: 90906951.0
(22) Anmeldetag: 30.04.1990
(51) Int. Cl.: H01S 3/094, H01S 3/042

(54) **FESTKÖRPERLASER MIT PUMP-LASERDIODEN**
LASER DIODE PUMPED SOLID STATE LASER
LASER SOLIDE A POMPAGE PAR DIODES LASER

(30) Priorität: 02.05.1989 DE 3914492
(43) Veröffentlichungstag der Anmeldung: 26.02.1992
(73) Patentinhaber: ADLAS GMBH & CO. KG, D-23569 Lübeck (DE)
(72) Erfinder: KORTZ, Hans-Peter, D-2409 Pansdorf (DE); SCRLAC, Walter, D-2067 Reinfeld (DE)
(74) Vertreter: Klunker . Schmitt-Nilson . Hirsch
(86) Internationale Anmeldenummer: EP9000693
(87) Internationale Veröffentlichungsnummer: WO9013930

(56) Entgegenhaltungen:
- DE-A- 2 542 652
- US-A- 4 719 631
- US-A- 4 756 002

## Beschreibung

Die Erfindung betrifft einen Festkörperlaser nach dem Oberbegriff des Anspruchs 1.

Die Verwendung von Laserdioden zur Bildung einer Pumplichtquelle für einen Festkörperlaser ist seit längerem bekannt. Die Laserdioden werden in einer geradlinigen Reihe auf einem Modul angeordnet, wobei das Modul parallel zur Längsachse des zylindrischen Laserstabs in geringem Abstand von dem Laserstrahl angeordnet wird (IEEE Journal of Quantum Electronics Vol. 24, Nr. 6, Juni 1988, Seiten 895 bis 911).

Die Verwendung von Laserdioden als Pumplichtquelle ist zwar wegen der relativ hohen Leistung des Laserlichtes (verglichen mit Leuchtdioden) vorteilhaft, wirft aber gleichzeitig gewisse Probleme hinsichtlich der Kühlung auf.

Eine Besonderheit der Pump-Laserdioden besteht darin, daß die Abstrahlkeule des Lichts nicht rotationssymmetrisch ist. Der Streuwinkel parallel zur Montagefläche ist wesentlich kleiner als senkrecht dazu:
Der Strahl läuft in der zur Montagefläche parallelen Richtung nicht so schnell auseinander (US-A-4 756 002). Senkrecht zur Montagefläche ist der Streuwinkel sehr groß. Dies führt bei der erwähnten bekannten Anordnung dazu, daß - wenn nicht eine Optik verwendet wird - ein Teil der Lichtenergie nicht in den Laserstab eingekoppelt wird und mithin verloren geht.

Zur Kühlung von Pump-Laserdiodenanordnung ist es bekannt (US-PS 4 719 631) mehrere, parallel zur Längsachse des Laserstabs stapelförmig angeordnete Dioden gemeinsam auf einer Keramik-Kühlplatte anzuordnen, wobei mehrere derartige Stapel um den Laserstab herum verteilt angeordnet sind. Die gestapelten Laserdioden bilden somit eine ebenfalls in Längsrichtung des Laserstabs verlaufende Anordnung.

Aus der DE-A 2 542 652 ist ein Festkörperlaser bekannt, bei dem das Festkörpermedium ein Hohlzylinder ist, in dem sich ein stabförmiges Diodenarray befindet, während es außen von einem rohrförmigen weiteren Diodenarray umgeben ist. Bei diesem Festkörperlaser nach dem Oberbegriff des Anspruchs 1 werden zwischen den beiden Diodenarrays einerseits und dem Festkörpermedium andererseits zwei Hohlräume für eine Kühlflüssigkeit gebildet. Die Dioden sind jeweils als Einzeldioden ausgebildet, alternativ ist jedoch die Möglichkeit angegeben, voll integrierte Diodenarrays zu verwenden, wobei die Dioden auch Laserdioden sein können.

Der Erfindung liegt die Aufgabe zugrunde, einen Festkörperlaser der eingangs genannten Art anzugeben, der durch die Möglichkeit einer effizienten Kühlung einen Betrieb bei hoher Leistung gestattet.

Diese Aufgabe wird durch die im Anspruch 1 angegebene Erfindung gelöst.

An den erfindungsgemäß vorgesehenen Kühlkanal können mühelos Zu- und Ableitungen angeschlossen werden. Die einzelnen Pumpmoduln können individuell gezielt gekühlt werden.

Vorzugsweise ist ein Pumpmodul, welches eine kreisförmige oder teilkreisförmige Anordnung von Laserdioden trägt, als Ring oder Teilring ausgebildet, und das Modul ist in einer Ebene senkrecht zur Längsachse des Laserstabs angeordnet. Da der Streuwinkel der Laserdioden senkrecht zu ihrer Montagefläche größer ist als der zur Montagefläche parallele Streuwinkel, kann unter Verzicht auf jegliche Optik auch bei relativ großem Abstand zwischen Pumpmodul und Laserstab eine große Lichtmenge in den Laserstab eingebracht werden.

Die Pumpmoduln können z.B. knapp halbkreisförmig ausgebildet sein. Sie können in Längsrichtung des Laserstabs stapelförmig angeordnet sein und/oder sie können winkelversetzt bezüglich des Laserstabs angeordnet sein, wobei jedoch stets jedes Modul in einer senkrecht zur Längsachse des Laserstabs verlaufenden Ebene orientiert ist.

Vorzugsweise werden die Laserdioden im inneren Randbereich des Pumpmoduls angeordnet. Damit ist im hinteren Bereich, d.h. im radial außenliegenden Bereich des Pumpmoduls genügend Platz zur Ableitung der Verlustleistung.

Vorzugsweise sieht die Erfindung vor, daß der Kühlkanal radial außen bezüglich der Laserdioden als Umfangskanal ausgebildet ist.

Ein derartiger Kühlkanal kann besonders einfach dadurch hergestellt werden, daß aus dem massiven Material (z.B. Kupfer) des Pumpmodul-Bodens eine Ringnut ausgefräst wird, und daß eine Abdeckplatte am Boden des Pumpmoduls angebracht wird, die die Ringnut nach unten abschließt.

Im folgenden werden Ausführungsbeispiele der Erfindung anhand der Zeichnung näher erläutert. Es zeigen
- Figur 1: eine schematisierte perspektivische Teilansicht eines Festkörperlasers, und
- Figur 2: eine Draufsicht auf einen Festkörperlaser.

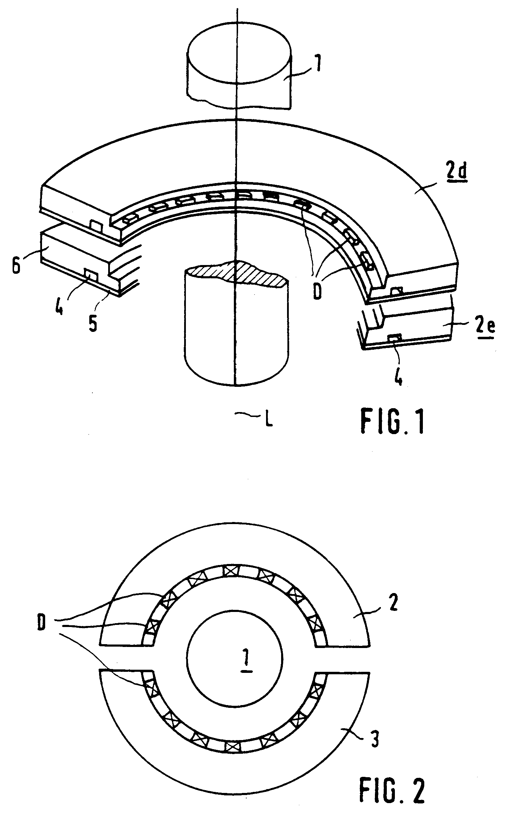

Nach Fig. 1 enthält ein Festkörperlaser einen aus einem üblichen Laserkristall hergestellten Laserstab 1. In verschiedenen, zur Längsachse L des Laserstabs 1 senkrecht verlaufenden Ebenen sind Pumpmoduln 2d und 2e und gegebenenfalls weitere, in der Zeichnung nicht dargestellte Pumpmoduln angeordnet.

Figur 2 zeigt in Draufsicht eine Anordnung eines Festkörperlasers, bei der ein Laserstab 1 von zwei winkelversetzt angeordneten Pumpmodulen 2 und 3 praktisch vollständig umgeben ist. Auch diese Pumpmodulen 2 und 3 können senkrecht zur Zeichenebene gestapelt angeordnet werden.

Die Pumpmodulen sind sämtlich identisch ausgebildet. Jedes Modul besteht aus einem massiven Materialblock, z.B. Kupferblock, in dessen Innenrandbereich eine Stufe ausgebildet ist, auf der eine Reihe von Laserdioden D angeordnet sind. Die Anschlüsse der Laserdioden sind in der Zeichnung nicht dargestellt.

Jedes Pumpmodul besteht aus einem massiven Teilring 6 und einem Bodenteil 5. In den Boden des Teilrings 6 ist eine Umfangsnut 4 eingefräst, die nach Anbringung der Bodenplatte 5 einen Kühlkanal für ein Kühlmittel, z.B. Wasser bildet. Das Kühlmittel wird über hier nicht dargestellte Anschlüsse durch die Umfangsnut bzw. Teilumfangsnut 4 geleitet.

In Abwandlung der oben beschriebenen Ausführungsformen können auch Radialnuten für das Kühlmittel vorgesehen sein.

Die Erfindung umfaßt in einem speziellen Aspekt auch eine geradlinige Anordnung von Modulen, deren Besonderheit darin besteht, daß in der Nähe der Laserdioden ein durchgehender Kühlkanal ausgebildet ist.

Durch die in den Fig. 1 und 2 dargestellte ringförmige Anordnung der Laserdioden D bleibt im äußeren Bereich der Pumpmodulen viel Raum zur Abstrahlung von Verlustwärme, die die Kühlung des Pumpmoduls fördert. Durch die relativ dichte Anordnung der Laserdioden D läßt sich eine große Lichtmenge in den Laserstab 1 einbringen.

Die Abstrahlkeule des von jeder einzelnen Diode D abgegebenen Laserlichts ist nicht-rotationssymetrisch, so daß die Haupt-Abstrahlleistung in einer Ebene liegt, die parallel zur Längsachse L des Laserstabs 1 verläuft. Deshalb gelangt praktisch das gesamte Laserlicht von den Dioden in den Laserstab 1.

## Patentansprüche

1. Festkörperlaser, mit einem Laserstab (1), einer aus in mindestens einer Reihe angeordneten Laserdioden (D) gebildeten Pumplichtquelle, die als Pumpmodul ausgebildet ist, und einer Kühleinrichtung zur Kühlung der Pumplichtquelle, **dadurch gekennzeichnet,** daß in dem Pumpmodul (2d, 2e; 2, 3) mindestens ein Kühlkanal (4) für ein Kühlmittel (z.B. Wasser oder Gas) ausgebildet ist.

2. Festkörperlaser nach Anspruch 1, **dadurch gekennzeichnet**, daß mindestens ein ringförmiges oder teilringförmiges Pumpmodul (2d, 2e; 2,3) vorgesehen ist, das in einer senkrecht zur Längsachse (L) des Laserstabs (1) verlaufenden Ebene angeordnet ist.

3. Festkörperlaser nach Anspruch 1 oder 2, **dadurch gekennzeichnet**, daß mehrere Pumpmodulen (2d, 2e) parallel zur Längsachse des Laserstabs (1) angeordnet sind.

4. Festkörperlaser nach einem der Ansprüche 1 bis 3, **dadurch gekennzeichnet**, daß mehrere teilringförmige Pumpmoduln (2, 3) winkelversetzt neben dem Laserstab (1) angeordnet sind.

5. Festkörperlaser nach einem der Ansprüche 1 bis 4, **dadurch gekennzeichnet**, daß der Kühlkanal radial außen bezüglich der Laserdiode (D) als Umfangskanal ausgebildet ist.

6. Festkörperlaser nach einem der Ansprüche 1 bis 5, **dadurch gekennzeichnet**, daß zur Bildung des Kühlmittelkanals (4) aus dem massiven Material, z.B. Kupfer, des Bodens des Pumpmoduls (1) eine Ringnut ausgebildet ist und daß an dem Boden eine Abdeckplatte (5) anliegt.

7. Festkörperlaser nach einem der Ansprüche 1 bis 6, **dadurch gekennzeichnet**, daß die einzelnen Laserdioden (D) derart angeordnet sind, daß ihre Hauptabstrahlleistung in einer Ebene liegt, die parallel zur Längsachse des Laserstabs (1) verläuft.

8. Festkörperlaser nach einem der Ansprüche 1 bis 7, **dadurch gekennzeichnet**, daß der Kühlkanal (4) in der Nähe der Laserdiodenreihe ausgebildet ist.

## Claims

1. A solid state laser having a laser rod (1), a pumped light source formed as a pumping module and comprising laser diodes (D) disposed in at least one row, and a cooling means for cooling the pumped light source, **characterized** in that at least one cooling duct (4) for a coolant (e.g. water or gas) is formed in the pumping module (2d, 2e; 2, 3).

2. The solid state laser of claim 1, **characterized** in that at least one ring-shaped or partial ring-shaped pumping module (2d, 2e; 2, 3) is provided which is disposed in a plane extending perpendicular to the longitudinal axis (L) of the laser rod (1).

3. The solid state laser of claim 1 or 2, **characterized** in that a plurality of pumping modules (2d, 2e) are disposed parallel to the longitudinal axis of the laser rod (1).

4. The solid state laser of any of claims 1 to 3, **characterized** in that a plurality of partial ring-shaped pumping modules (2, 3) are offset at an angle beside the laser rod (1).

5. The solid state laser of any of claims 1 to 4, **characterized** in that the cooling duct is formed as a circumferential channel on the radial outside with respect to the laser diode (D).

6. The solid state laser of any of claims 1 to 5, **characterized** in that an annular groove is formed from the massive material, e.g. copper, of the bottom of the pumping module (1) to form the coolant duct (4), and a cover plate (5) lies against the bottom.

7. The solid state laser of any of claims 1 to 6, **characterized** in that the individual laser diodes (D) are disposed such that their main radiating power lies in a plane extending parallel to the longitudinal axis of the laser rod (1).

8. The solid state laser of any of claims 1 to 7, **characterized** in that the cooling duct (4) is formed in the vicinity of the row of laser diodes.

## Revendications

1. Laser solide, comportant un barreau de laser (1), une source de lumière de pompage qui est formée de diodes laser (D) disposées en au moins une rangée et qui est conçue en tant que module de pompage, ainsi qu'un dispositif de refroidissement, pour refroidir la source de lumière de pompage, caractérisé par le fait que dans le module de pompage (2d, 2e; 2,3) est prévu au moins un canal de refroidissement (4) pour un réfrigérant (par exemple eau ou gaz).

2. Laser solide selon la revendication 1, caractérisé par le fait qui est prévu au moins un module de pompage, en forme d'anneau ou d'anneau partiel (2d, 2e ; 2,3), qui est disposé dans un plan orienté perpendiculairement à l'axe longitudinal (L) du barreau de laser (1).

3. Laser solide selon la revendication 1 ou 2, caractérisé par le fait que plusieurs modules de pompage (2d, 2e) sont disposés parallèlement à l'axe longitudinal du barreau de laser (1).

4. Laser soluble selon l'une des revendications 1 à 3, caractérisé par le fait que plusieurs modules de pompage (2, 3), en forme d'anneau partiel, sont disposés, décalés angulairement, auprès du barreau de laser (1).

5. Laser solide selon l'une des revendications 1 à 4, caractérisé par le fait que le canal de refroidissement est conçu sous forme de canal périphérique situé à l'extérieur des diodes laser (D), dans une direction radiale par rapport à celles-ci.

6. Laser solide, selon une des revendications 1 à 5, caractérisé en ce que le canal de refroidissement (4) est constitué d'une gorge annulaire formée dans le matériau massif, par exemple du cuivre, constitutif de la base du module de pompage, et en ce qu'une plaque de couverture (5) est posée contre cette base.

7. Laser solide, d'après une des revendications 1 à 6, caractérisé en ce que les diodes laser (D) sont disposées de telle façon que l'émission principale du rayonnement soit comprise dans un plan parallèle à l'axe longitudinal du barreau laser (1).

8. Laser solide d'après une des revendications 1 à 7, caractérisé en ce que le canal de refroidissement (4) est disposé au voisinage de la rangée de diodes laser.
